(19) Europäisches Patentamt
European Patent Office
Office européen des brevets

(11) Publication number: **0 222 778 B1**

(12) **EUROPEAN PATENT SPECIFICATION**

(45) Date of publication of patent specification: **16.02.94**

(51) Int. Cl.5: **A01N 59/12**, A61K 33/18, C11D 3/48

(21) Application number: **86901753.3**

(22) Date of filing: **13.03.86**

(86) International application number: **PCT/AU86/00069**

(87) International publication number: **WO 86/05359 (25.09.86 86/21)**

(54) **ANTISEPTIC COMPOSITIONS.**

(30) Priority: **13.03.85 AU 9677/85**

(43) Date of publication of application: **27.05.87 Bulletin 87/22**

(45) Publication of the grant of the patent: **16.02.94 Bulletin 94/07**

(84) Designated Contracting States: **AT BE CH DE FR GB IT LI LU NL SE**

(56) References cited:
**AU-B- 2 412 /51**
**AU-B-40 487 /68**
**DE-A- 1 593 155**
**GB-A- 2 084 464**
**US-A- 3 751 565**
**US-A- 3 777 022**
**US-A- 4 206 204**
**US-A- 4 288 428**

(73) Proprietor: **GLUCK, Bruno Anthony**
**20 King Avenue**
**Balgowlah, NSW 2093(AU)**

(72) Inventor: **GLUCK, Bruno Anthony**
**20 King Avenue**
**Balgowlah, NSW 2093(AU)**

(74) Representative: **Wilkinson, Stephen John et al**
**Stevens, Hewlett & Perkins**
**1 St. Augustine's Place**
**Bristol BS1 4UD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 222 778 B1

Rank Xerox (UK) Business Services
(3.10/3.09/3.3.3)

## Description

### TECHNICAL FIELD

The present invention relates to antiseptic compositions which have both a rapid and residual antimicrobial and antiviral action. More particularly, the invention relates to compositions containing an iodophor which has a rapid action together with one or more agents having a residual antibacterial and/or antiviral action.

The compositions of the invention find use in medical applications such as antiseptic skin cleansers, antiseptic solutions for use under plaster casts and dressings, antiseptic lotions for skin disinfection, e.g. prior to surgery or first aid procedures, preoperative skin wash and shower preparations, shampoos, medicated after-shave preparations, tinea preparations, hospital laundry rinses, nappy (diaper) sanitisers and surface sprays.

Agricultural/veterinary applications in which the compositions of the invention find use include preparations for treatment and prophylaxis of foot rot and animal shampoos.

It has been surprisingly discovered that some synergism occurs between the iodophor and the residual antibacterial antiviral agent.

### BACKGROUND ART

There is continuous demand by surgeons and members of the medical profession for a practical method of handcleansing other than using only soap and water, but which would provide additionally to the cleansing of the skin, rapid disinfection combined with residual protection against immediate re-infection.

A desirable hand and skin cleansing composition would be one which would combine, additionally to the effective cleansing of the skin, rapid disinfection and protection against subsequent re-infection.

Iodine, because of its exceptional biocidal properties has long been a favoured germicidal ingredient for skin cleansing compositions.

The advantages of iodine in combination with iodine carriers resulting in complex formation, having available iodine as active biocidal constituent, over precious iodine preparations having the iodine solubilised by forming the triiodide with an alkali or hydrogen iodide, such as tincture of iodine or Lugol's solution, are well known and as exemplified in US Patent No. 3 777 022.

Iodine, in its complex formation, especially in combination with polyvinylpyrrolidone, known as povidone-iodine, has, because of its rapid action and wide microbicidal spectrum been used as the active germicidal ingredient in various brands of antiseptic skin cleansers for preoperative scrub-up and general ward use.

Despite the great advantages of these antiseptic hand and skin cleansing products over others, not containing iodine, they suffer from the disadvantage, that once used, they leave the skin with no residual prophylactic protection against re-infection.

Other germicides such as the quaternary ammonium compounds or chlorhexidine salts, although not quite as fast acting as the iodophors, have found wide application in various antiseptic preparations. AU-B-436196 relates to germicidal detergent compositions comprising quaternary ammonium germicides and non-ionic detergents. The compositions may contain an iodine complex. DE-A-1593155 discloses chlorhexidine compositions which may additionally contain iodine in the form of an iodophor or as a complex with the chlorhexidine.

Although such other germicides do not possess the wide microbicidal spectrum of the iodophors, and are not equally active against the various strains of Gram-positive and negative micro-organisms, they do provide a persistent antibacterial effect on the skin after application.

Bacteriostats form a third group of antimicrobial agents which have found wide use in skin and handwashing preparations. They have a high affinity to the skin and confer remanent antibacterial properties, which continue to build up on repeated application, eventually conferring a complete shield on the skin against re-infection. They also reduce the resident bacterial flora of the skin to a minimum, which is not removed by the conventional antibacterial agents mentioned previously.

The importance of combining a fast acting broad spectrum biocide such as the iodine complex lacking persistant action after application with an agent providing residual and continuous anti microbial activity is self evident.

This is true in a wider sense of all applications where rapid disinfection of any surface is required and persistent protection against re-infection is desired, even if only for a limited time.

This is of special importance for medical personnel when an antiseptic hand and skin cleansing preparation combining an iodine complex with a bacteriostat with residual properties allows the immediate disinfection of the skin and additionally provides an antibacterial shield against possible re-infection.

The provision of an antiseptic composition combining even, rapid biocidal action over practically the complete microbiological spectrum, combined with remanent antibacterial effectiveness present therefore an important advance in the technique of hand and skin sanitation and disinfection of surfaces in general.

Various attempts have been made to combine bacteriostats with iodine in disinfectant solutions. Most of these have been unsuccessful, as due to possible reaction between iodine and the bacteriostat component of the preparation shown by considerable loss of iodine occurring on standing. Such attempts have been described in U.S. Patent No. 3 777 022 and Canadian Patent No. 729 597.

DESCRIPTION OF THE INVENTION

The present invention provides an antiseptic composition comprising at least one iodine complex and at least one water-insoluble bacteriostat together with a pharmaceutically, veterinary, or agriculturally acceptable carrier or diluent therefor.

In the specification and claims, the term iodine complex is a complex of iodine with polyvinylpyrrolidone known also as povidone-iodine, or nonionic surfactants of the general formula

$R(CH_2CH_2O)_xH$

where R represents and alkylphenol, a fatty alcohol or acid residue and x is 6 to 100, and preferably 7 to 15, or polycondensates of ethylene oxide and propylene oxide of a molecular weight of not less than 1000, preferably between 2500 and 4000. Such complexes have because of their rapid action and wide microbicidal spectrum become the active germicidal ingredient in various brands of antiseptic skin cleansers for preoperative scrub-up, general medical and household antiseptic preparations, veterinary uses and disinfectant cleansers for the food and allied industries. Iodine complexes with amphoteric surface active agents and quaternary ammonium compounds are also included in the definition.

The preferred complex of iodine and polyvinylpyrrollidorl has 10% available iodine and a K-value of 30.

Other suitable iodine complexes include nonylphenoxy-(ethyleneoxy)-iodine, polyethyleneoxypolypropyleneoxy-iodine and undecoylinium-chloride-iodine, as well as the other commercially available products.

Suitable bacteriostats which may be employed in compositions of the invention include halogenated hydroxydiphenyl derivatives or halogenated salicyl and carbanilides.

Examples of these bacteriostats are those of the formula

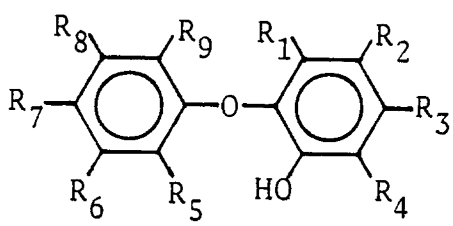

I

wherein each of $R_1$ to $R_9$ may be hydrogen, halogen, lower alkyl, haloloweralkyl, lower alkoxy, allyl, cyano, amino or acetyl and the O-acyl derivatives thereof provided that at least one of $R_1$ to $R_9$ is halogen;

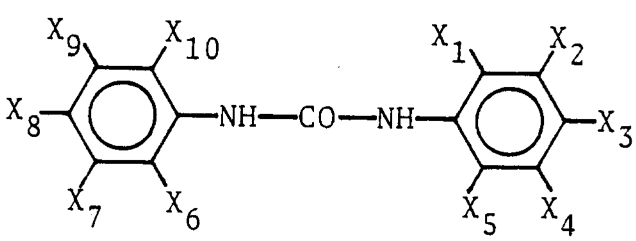

II

wherein each of $X_1$ to $X_{10}$ may be hydrogen, halogen, haloalkyl, nitro or alkoxy provided that at least one of $X_1$ to $X_9$ is halogen;

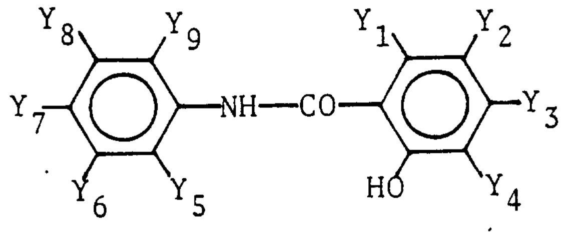

III

wherein each of $Y_1$ to $Y_9$ is hydrogen or halogen, provided that at least two of $Y_1$ to $Y_9$ are halogen: and

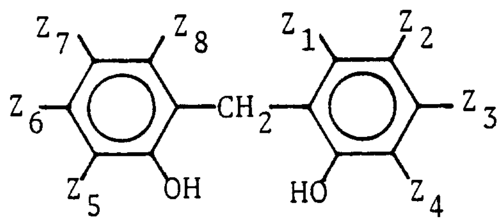

wherein each of Z, to $Z_8$ may be hydrogen or halogen provided that there is at least two halogen substituents on each Phemyl ring.

Such compounds are disclosed in Australian Patents Nos. 200 868, 209 986, 236 460, 273 941, 278 661 and 283 658 and in US Patent Nos. 2 250 840, 2 967 885, 3 254 121, 3 057 920 and 3 064 048.

Examples of the preferred bacteriostats include 2,4,4'-trichloro-2'-hydroxydiphenyl ether (triclosan); 2,2'-dihydroxy-3,3',5,5',6,6'-hexachlorodiphenylmethane systematic name (hexachlorophene); 3,5,4'-tribromosalicylanilide (tribromsalan) and 3,4,4'-trichloro- or 3-trifluoromethyl-4,4'-dichloro-carbanilides (triclocarban and cloflucarban). Triclosan is preferred for carrying out the invention.

It is preferred that the compositions of the invention contain a greater amount by weight of available iodine than of bacteriostat.

It is preferred that the iodine complexes are present in the compositions of the invention so as to provide from 0.01 to 5.0% w/v especially 0.35 to 2.0% w/v available iodine. It is preferred that the compositions contain 0.01 to 3.0% w/v, especially 0.5 to 2.0% w/v of the bacteriostats.

It is further preferred that if one or more surfactants should be part of the composition according to the invention, one at least should be a nonionic surface agent.

Antiseptic compositions of the invention remain stable at temperatures below 25 °C over considerable time of up to two years in the absence of light.

The combined biocidal activity of the available iodine and the bacteriostat is greater against certain bacteria than could be expected from the values of the biocidal strength of the separate iodine or bacteriostat components added together.

The cumulative effect of the iodine complex and bacteriostat is shown by minimum inhibitory tests in vitro by serial dilutions using Difco AOAC medium and illustrated by the following table:

Triclosan 1.0% w/v in a detergent solution

| Test Cultures | Dilutions | | | | |
|---|---|---|---|---|---|
| | 1/25 | 1/50 | 1/100 | 1/200 | 1/400 |
| E.coli 6.4 x $10^9$ | + | + | + | + | + |
| Staph.aureus 6.3 x $10^9$ | + | + | + | + | + |
| P valgaris 6.0 x $10^9$ | + | + | + | + | + |
| Ps.aeruginosa | + | + | + | + | + |

Povidone-Iodine 0.75% av.iodine in same detergent solution

| | 1/25 | 1/50 | 1/100 | 1/200 | 1/400 |
|---|---|---|---|---|---|
| E.coli 6.4 x $10^7$ | - | + | + | + | + |
| Staph.aureua 6.3 x $10^9$ | - | - | - | - | - |
| P. Vulgaris 6.0 x $10^9$ | + | + | + | + | + |
| Pr.aeruginosa | + | + | + | + | + |

Povidone-Iodine 0.5% and Triclosan 1.0% w/v in same

| | 1/25 | 1/50 | 1/100 | 1/200 | 1/400 |
|---|---|---|---|---|---|
| E.coli 6.4 x $10^9$ | - | - | - | - | - |
| Staph.aureus 6.3 x $10^9$ | - | - | - | - | - |
| Ps.vulgaris 6.0 x $10^9$ | - | - | - | - | - |
| PS.aeruginosa 6.0 x $10^9$ | - | - | + | + | + |

+ = bacterial growth

- = no growth

Where surface active agents are used to supply the necessary detergency in compositions where detergency is needed, they must not only be saturated compounds but also be stable in solution having a pH less than 6 to avoid separating from solution on prolonged standing. Hand cleansers of the invention retain satisfying foaming properties at the pH of the composition. Although the pH of the composition can range for practical reason between 2 to 6, it is preferable to select a pH between 4 to 5 for skin cleansers and antiseptics and around 2 for surface disinfectant cleansers.

Suitable surface active agents are nonionics such as the nonylphenolpolyethoxy condensates, the alkali, ammonium and triethanolamine salts of phenoxy- or alkyloxypolyoxyethylene sulfonates, or polycondensates with ethylene oxide and propylene oxide, used on their own, or mixtures thereof.

It is advantageous to use, in conjunction with the surfactants, foaming agents such as the fatty acid diethanolamides or an alkyldimethylamine oxide such as a lauryldimethylamine oxide, lauryldiethanolamide being preferred.

The bacteriostat is suitably added in a common solvent such as ethanol, isopropanol or propylene glycol or directly, provided the composition has sufficient solubilising power to keep the bacteriostat in solution.

According to the various compositions and their different applications, various ingredients will have to be added to give the composition additional desired properties. Care has to be taken in the choice of such ingredients to assure that they will be compatible with the iodine complex and not affect its stability.

If desired, glycerine or other emollients commonly used in hand and skin cleansing preparations can be added, provided they are compatible with the iodine complex and bacteriostat used in the composition.

MODES FOR CARRYING OUT THE INVENTION

The following examples illustrate preferred embodiments of the present invention. They should not be construed as limiting the claims hereto. The ingredients are combined by standard cold mixing processes.

EXAMPLE 1

| ANTISEPTIC SKIN AND HAND CLEANSER | |
|---|---|
| iodine complex * | 4.0g |
| nonionic surfactants ** | 5.0g |
| sodiumlaurylethoxysulfate *** | 20.0g |
| lauryldimethylamineoxide **** | 5.0g |
| glycerol | 1.0g |
| trichlocarban | 2.0g |
| propanol | 5.0mL |
| phosphoric acid to adjust to | pH 4.5 |
| water to make | 100.0mL |

* (Antarox VRO 20 available from GAF Corp. USA)
** (Antarox CO 730 available from GAF Corp. USA)
*** (Empigen 5Q25 available from Albright & Wilson Inc.)
**** (Empigen OB available from Albright & Wilson Inc.)

EXAMPLE 2

| SANITISING CLEANSER | |
|---|---|
| povidone-iodine, containing 10.0% w/v available iodine | 7.5g |
| sodium salt of sulphonated alkylphenoxypoly (ethyleneoxy)ethanol * | 20.0g |
| lauryldiethanolamide | 2.0g |
| glycerol | 1.0g |
| triclosan | 0.5g |
| isopropanol | 10.0mL |
| phosphoric acid to adjust to | pH 4.5 |
| water to make | 100.0mL |

* (Fenopan Co., available from GAF Corp. USA)

The bacteriostat is dissolved in the isopropanol prior to mixing with the other ingredients.

EXAMPLE 3

| ANTISEPTIC SOLUTION | |
|---|---|
| povidone-iodine, containing 10.0% w/v available iodine | 10.0g |
| ethanol isopropanol mixture 2:1 | 40.0mL |
| tribromsalan | 0.25g |
| nonionic surfactant | 0.50g |
| phosphate-citrate buffer to adjust to | pH 4.5 |
| water to volume | 100.00mL |

The triclosan is dissolved in the alcohol mixture prior to mixing.

EXAMPLE 4

| ANTISEPTIC TINCTURE | |
|---|---|
| povidone-iodine, containing 10.0% w/v available iodine | 5.0g |
| lanogel 61 * | 0.25g |
| ethanol | 70.00mL |
| triclosan | 0.25g |
| phosphate-citrate buffer to adjust to | pH 4.5 |
| Water to volume | 100.0mL |

* Nonionic lanolin derived surfactant (Amerchol Corp. New Jersey USA)

EXAMPLE 5

| PREOPERATIVE SKIN WASH & SHOWER PREPARATION | |
|---|---|
| povidone-iodine, containing 10.0% w/v available iodine | 10.0g |
| nonionic surfactant | 5.0g |
| sodium laurylethoxysulfate* | 30.0g |
| Coconutdiethanolamide | 2.0g |
| propylene glycol | 10.0mL |
| triclosan | 1.0g |
| phosphoric acid to adjust to | pH 4.5 |
| Water to volume | 100.0mL |

* (Empigen 5Q25 available from Albright & Wilson Inc.)

The PVP-I and the sodium lauryl and coconutdiethanolamide were dissolved in water and triclosan dissolved in the propylene glycol was added slowly with efficient stirring. The pH was adjusted and the composition adjusted to final volume with water.

EXAMPLE 6

| MEDICATED AFTER SHAVE | |
|---|---|
| povidone-iodine, containing 10.0% w/v available iodine | 0.5g |
| Glucan E 10* | 4.0g |
| Glucan P 20* | 3.0g |
| ethanol | 45.0mL |
| propylene glycol | 10.0mL |
| triclosan | 0.01g |
| triclocarban | 0.01g |
| glycerin | 2.5g |
| phosphate-citrate buffer to | pH 5.5 |
| perfume, colour | q.s |
| water to volume | 100.0mL |

* [nonionicpropoxylated glucose derivate (Amerchol Corp. New Jersey, U.S.A. )]

EXAMPLE 7

| TINEA TREATMENT | |
|---|---|
| povidone-iodine, containing 10.0% w/v available iodine | 10.0g |
| polyethylene glycol 400 | 60.0g |
| polyethylene glycol 4000 | 25.0g |
| triclosan | 0.5g |
| water | 4.0g |

The mixture of the polyethylene glycols was heated to 45°C and the triclosan dissolved with slight stirring followed by the PVP-I. The clear melt was left to cool slowly until a creamy consistency was obtained. The ointment like product is then ready for packing.

EXAMPLE 8

| TEAT DIP - for mastitis prophylaxis | |
|---|---|
| iodine complex * | 2.5g |
| glycerin | 4.0g |
| propylenglycol | 3.0mL |
| triclosan | 0.5g |
| phosphate-citric buffer to | pH 4.0 |
| water to volume | 100.0mL |

* (Antarox VRO 20 available from GAF Corp. USA)

EXAMPLE 9

| DOG SHAMPOO | |
|---|---|
| iodine complex * | 5.0g |
| nonionic surfactant | 5.0g |
| sodium lauryl ethoxy sulfate | 20.0g |
| coconutdiethanolamide | 2.0g |
| propylene glycol | 5.0g |
| hexachlorophene | 0.5g |
| phosphoric acid to adjust to | pH 4.0 |
| water to volume | 100.00mL |

* (Antarox VRO 20 available from GAF Corp. USA)

EXAMPLE 10

| BATH PREPARATION FOR PRE-OP AND DERMATOLOGICAL CONDITIONS | |
|---|---|
| iodine complex * | 10.0g |
| nonionic surfactant | 10.0g |
| triclosan | 1.75g |
| sodium laurylethoxy sulphate | 40.0g |
| coconutdiethanolamide | 5.0g |
| citric acid to adjust to | pH 4.5 |
| Water to make | 100.00mL |

* (Antarox VRO 20 available from GAF Corp. USA)

COMPARATIVE EXAMPLE 1

An aqueous cleansing solution containing 7.5% povidone-iodine (0.94% w/v available iodine) was divided into two parts, whereby to one part thereof was added 2.0% of triclosan.

After standing for two years at ambient temperatures both products showed identical small losses of available iodine, demonstrating that no reaction between the bacteriostat and the iodine had occurred.

**Claims**

**1.** An antiseptic composition comprising at least one iodine complex and at least one water insoluble bacteriostat together with a pharmaceutically, veterinary, or agriculturally acceptable carrier or diluent therefor.

**2.** A composition as defined in claim 1, wherein the iodine complex is a complex between iodine, and polyvinylpyrrolidone or a polycondensate of ethyleneoxide with propyleneoxide, alkylphenols, fatty alcohols, fatty acids, quaternary ammonium compounds or amphoterics surface active agent.

**3.** The composition as defined in claim 2, wherein the iodine complex is povidone-iodine.

**4.** A composition as defined in claim 1, wherein the bacteriostat is a halogenated hydroxydiphenyl derivative, a halogenated salicylanilide or a halogenated carbanilide.

**5.** A composition as defined in claim 4, wherein the bacteriostat is 2,4,4'-trichloro-2'-hydroxydiphenyl ether, 2,2'dihydroxy-3,3',5,5',6,6'-hexachlorodiphenylmethane, 3,5,4'-tribromosalicylanilide, 3,4,4'-trichlorocarbanalide, or 3-trifluoromethyl-4,4'-dichlorocarbanilide.

**6.** A composition as defined in claim 4, wherein the bacteriostat is 2,4,4'-trichloro-2'-hydroxydiphenyl ether.

**7.** A composition as defined in claim 1, further comprising a saturated surface active agent stable in solution having a pH of less than 6.

**8.** A composition as defined in claim 7, wherein the surface active agent is a nonylphenolpolyethoxy condensate or an alkali ammonium or triethanolamine salt of a phenoxy- or alkyloxypolyoxyethylene sulfonate or a polycondensate of ethylene oxide and propylene oxide or a mixture of two or more thereof.

**9.** A composition as defined in claim 7, further comprising a foaming agent.

**10.** A composition as defined in claim 9, wherein the foaming agent is a fatty acid diethanolamide or alkyldimethylamine oxide.

**11.** A composition as defined in claim 1, having a pH-value less than 7.

**Patentansprüche**

**1.** Antiseptische Zusammensetzung, umfassend wenigstens einen Iodkomplex und wenigstens ein wasserlösliches Bakteriostatikum zusammen mit einem pharmazeutisch, tierärztlich oder landwirtschaftlich annehmbaren Träger oder Verdünnungsmittel dafür.

**2.** Zusammensetzung wie in Anspruch 1 definiert, in welcher der Iodkomplex ein Komplex zwischen Iod und Polyvinylpyrrolidon oder einem Polykondensat von Ethylenoxid mit Propylenoxid, Alkylphenolen, Fettalkoholen, Fettsäuren, quaternären Ammoniumverbindungen oder einem amphoteren oberflächenaktiven Mittel ist.

**3.** Zusammensetzung wie in Anspruch 2 definiert, in welcher der Iodkomplex Povidon-Iod ist.

**4.** Zusammensetzung wie in Anspruch 1 definiert, in welcher das Bakteriostatikum ein halogeniertes Hydroxydiphenylderivat, ein halogeniertes Salicylanilid oder ein halogeniertes Carbanilid ist.

**5.** Zusammensetzung wie in Anspruch 4 definiert, in welcher das Bakteriostatikum 2,4,4'-Trichlor-2'-hydroxydiphenylether, 2,2'-Dihydroxy-3,3',5,5',6,6'-hexachlordiphenylmethan, 3,5,4'-Tribromsalicylanilid, 3,4,4'Trichlorcarbanilid oder 3-Trifluormethyl-4,4'-dichlorcarbanilid ist.

**6.** Zusammensetzung wie in Anspruch 4 definiert, in welcher das Bakteriostatikum 2,4,4'-Trichlor-2'-hydroxydiphenylether ist.

**7.** Zusammensetzung wie in Anspruch 1 definiert, welche weiter ein gesättigtes oberflächenaktives Mittel enthält, das in einer Lösung mit einem pH kleiner als 6 stabil ist.

**8.** Zusammensetzung wie in Anspruch 7 definiert, in welcher das oberflächenaktive Mittel ein Nonylphenolpolyethoxy-Kondensat oder ein Alkali-, Ammonium- oder Triethanolaminsalz eines Phenoxy- oder Alkyloxypolyoxyethylensulfonats oder ein Polykondensat von Ethylenoxid und Propylenoxid oder ein Gemisch zweier oder mehrerer davon ist.

**9.** Zusammensetzung wie in Anspruch 7 definiert, welche weiter einen Schaumbildner enthält.

**10.** Zusammensetzung wie in Anspruch 9 definiert, in welcher der Schaumbildner ein Fettsäurediethanolamid oder Alkyldimethylaminoxid ist.

**11.** Zusammensetzung wie in Anspruch 1 definiert mit einem pH-Wert kleiner als 7.

**Revendications**

**1.** Composition antiseptique comprenant au moins un complexe iodé et au moins un bactériostatique insoluble dans l'eau, ainsi qu'un véhicule ou diluant acceptable en pharmacie, en médecine vétérinaire ou en agriculture.

**2.** Composition selon la revendication 1, dans laquelle le complexe iodé est un complexe formé entre l'iode et la polyvinylpyrrolidone ou un polycondensat d'oxyde d'éthylène et d'oxyde de propylène, des alkylphénols, des alcools gras, des acides gras, des composés ammonium quaternaire ou des agents tensioactifs amphotères.

**3.** Composition selon la revendication 2, dans laquelle le complexe iodé est de la polyvidone-iode.

**4.** Composition selon la revendication 1, dans laquelle le bactériostatique est un dérivé hydroxydiphényle halogéné, un salicylanilide halogéné ou un carbanilide halogéné.

**5.** Composition selon la revendication 4, dans laquelle le bactériostatique est l'éther 2,4,4'-trichloro-2'-hydroxydiphénylique, le 2,2'-dihydroxy-3,3',5,5',6,6'-hexachlorodiphénylméthane, le 3,5,4'-tribromosalicylanilide, le 3,4,4'-trichlorocarbanilide ou le 3-trifluorométhyl-4,4'-dichlorocarbanilide.

6. Composition selon la revendication 4, dans laquelle le bactériostatique est l'éther 2,4,4'-trichloro-2'-hydroxydiphénylique.

7. Composition selon la revendication 1, comprenant en outre un agent tensioactif saturé stable en solution, de pH inférieur à 6.

8. Composition selon la revendication 7, dans laquelle l'agent tensioactif est un condensat nonylphénolpolyéthoxy ou un sel d'alcalin, d'ammonium ou de triéthanolamine d'un sulfonate de phénoxy- ou alkyloxypolyoxyéthylène ou un polycondensat d'oxyde d'éthylène et d'oxyde de propylène ou un mélange de deux ou plusieurs de ces substances.

9. Composition selon la revendication 7, comprenant en outre un agent moussant.

10. Composition selon la revendication 9, dans laquelle l'agent moussant est un diéthanolamide d'acide gras ou un oxyde d'alkyldiméthylamine.

11. Composition selon la revendication 1, de pH inférieur à 7.